# EUROPEAN PATENT SPECIFICATION

(11) **EP 4 369 352 B1**
(45) Date of publication and mention of the grant of the patent: **16.09.2026**
(21) Application number: 22206957.7
(22) Date of filing: 11.11.2022
(51) Int. Cl.: G16H 20/40, G16H 30/40

(54) **SYSTEM FOR VIRTUAL REALITY-BASED PREOPERATIVE SURGICAL PLANNING**
SYSTEM ZUR PRÄOPERATIVEN CHIRURGISCHEN PLANUNG AUF DER BASIS VON VIRTUELLER REALITÄT
SYSTÈME DE PLANIFICATION CHIRURGICALE PRÉOPÉRATOIRE BASÉE SUR LA RÉALITÉ VIRTUELLE

(43) Date of publication of application: 15.05.2024
(73) Proprietor: MedicalVR B.V., 2153 NB Nieuw-Vennep (NL)
(72) Inventor: Hordijk, Chris, 2153NB Nieuw-Vennep (NL)
(74) Representative: WENDE IP GbR

(56) References cited:
- US-A1- 2021 145 521

## Description

The present invention relates to a system for preoperative surgical planning in virtual reality environments, and more specifically to a system for visualising organs for planning of resections of tumors prior to surgery and for providing an immersive view into anatomical structures of a patient.

Over the past few years, virtual reality (VR) devices and software tools have been gaining popularity and have been shown to be a valuable instrument improving the understanding of anatomy of patients by surgeons. VR assessment platforms include more features and functions than existing 2D or 3D planning software that facilitate an immersive and interactive manipulation, realistic in-depth perception and visualization of complex relationships of anatomic structures. Surgeons therefore gain more realistic and detailed insights into the anatomy of the subject penitent.

Moreover, automated imaging algorithms could potentially create a more efficient planning by enabling visualization of the anatomic structures of interest. This may further be improved by developing a combined artificial intelligence (AI)-based and immersive 3D-VR platform as a supplementary preoperative planning tool to traditional CT imaging.

In particular, in surgeries such as thoracoscopic lung segmentectomies which are generally more complex than thoracoscopic lobectomies, identifying intraparenchymal planes and ligation of target branches of pulmonary arteries, veins, and bronchi can be more challenging. This is partly due to the existence of many anatomical variations and abnormalities in pulmonary vascular anatomy.

The success of pulmonary segmentectomies may therefore largely depend on the surgeon's preoperative understanding of the patient's anatomy. Although conventional computed tomography (CT) imaging techniques are the gold standard for preoperative planning of anatomical (sub)lobar resections however the identification of segmental borders and segmental branches of arteries, veins, and bronchi becomes more challenging when conventional 2-dimensional (2D) CT techniques are employed.

US 2021/0145521 A1 describes a system for presenting medical imaging data in an interactive virtual environment reality. The system comprises a head mounted display that: determines that a transparent 3D object overlaps a 3D model of a portion of anatomy based on a medical imaging scan; sets values for pixels corresponding to portions of the 3D model not occluded by the transparent object by performing a shading operation; sets values for pixels corresponding to portions of the 3D model at the boundary of the transparent object to intensity values taken from the medical imaging data; displays the 3D model with an exterior surface shaded to evoke a 3D object, and internal surfaces at the boundaries of the transparent object not shaded to preserve details embedded in the medical imaging data.

It is thus an object of the present invention to provide for a system for visualising organs for planning of segmentectomy and/or lobectomy procedures that provides for an accurate and a patient-specific insight into the organs.

This object is achieved by the system according to independent claim 1. Further advantageous embodiments can be realized by the systems according to the dependent claims.

According to the present invention, a system for visualizing organs for planning of resections of tumors, including details to the segmentation and further data related to a patient is provided. In addition, the system provides for an immersive view into anatomical structures of the patient. The system comprises:
- a control module comprising data storage devices and processing resources configured to store, access and execute computer-readable program instructions;
- a server communicating over a communication network with the control module;
- a user interaction module comprising a multiplayer module configured to facilitate a real-time interaction of a user with the system;
- a user input module configured to control and to receive input information from the user, wherein the user input module comprises a plurality of input devices, a virtual reality input module for receiving input in a virtual reality environment, and a virtual reality control module for conducting operations based on the information received from the virtual reality input module; and
- an input/output module comprising a rendering module, a motion tracking device, a 3D-display, a 2D-display and a head-mounted display for an immersive viewing;
- the user interaction module, the user input module and the input/output module communicating over a communication interface with each other and with the control module;
- the control module configured to receive image data and processed data from the server, and to store the same in the data storage devices, wherein the image data comprise 2D computed tomography images of the anatomical structures, and wherein the processed data comprise segmentation data representative of segments of the anatomical structures;
- the user interaction module configured to create a visualization of the anatomical structures in 2D and 3D based on the image data and the processed data received from the control module, and to edit the created 2D and 3D visualizations based on the input information received from the user input module;
- the input/output module configured to display the visualizations on the corresponding 2D-display and the 3D-display, and to provide an immersive view into the anatomical structures of the patient through the head-mounted display, wherein the immersive view is generated from the image data and the processed data, preferably using a raymarching technique;
- the input/output module further configured to edit, using the rendering module, the 2D visualization, the 3D visualization and the immersive view based on the information received from the user interaction module, the user input devices, the virtual reality input module and the motion tracking device.

The system advantageously enables a better understanding of the anatomical structures of a subject patient and provides for a detailed, authentic immersive view of the anatomical structures and segmentations thereof. The system further advantageously allows a real-time interaction with the displayed images (e.g. to modify the image size, colour, orientation and the view angle). In addition, the system enables the manipulation of the displayed images in real time while also facilities a dynamic interaction with other users, e.g. by way of a voice chat capability to simulate a live session together with other remote users. In particular, the system allows an immersive visualization for the user of anatomic structures such as the lung, but also other tissue or organ structures. By having these immersive and "deep dive" visualizations at hand prior to an operation, a surgery can be planned more reliably by one or several surgeons resulting into performing the surgery in a more accurate and controlled manner. Due to the provision of the immersive visualizations during the pre-operative planning viewing 3D-renders (or 3D models) of the subject anatomical structures, it is possible for the surgeon to have a far better understanding of the anatomy of the specific patient, while planning the surgery. This in turn reduces the risk of unexpected errors or issues while performing a surgery and provide for a better orientation and less surprises during the surgery. As a possible outcome, the resection planned with the system according to the present invention will be more accurate and less invasive, because of the advanced and far more accurate planning e.g. less tissue of organs must be resected. This is especially very beneficial in tumor resections in lung tissue, but also in other organs such as liver or tissue structures like the brain. Generally speaking, the system is applicable for all kind of tissue of a patient.

In particular, the system comprises modules for viewing 2D and/or 3D visualisations on a mobile device, e.g. on a mobile phone, a tablet and/or a web browser. In this way, the system advantageously provides for mobile visualizations of the 3D models of the anatomical structures.

The system may further comprise a remote display device (e.g. including a 2D-dispay and a 3D-display) that is configured to display the 2D and/or 3D visualisations, thereby enabling surgeons to view the 2D-scans and/or 3D models during a surgical procedure.

In particular, the system comprises a control module that is configured to communicate over a communication network with a server for receiving medical images, scan data and other information therefrom. The scan data may include 2D computed tomography scan data, manually created 3D segmentations and 3D segmentation data that are created by artificial intelligences (AI)-based algorithms.

The control module comprises the data storage devices for storing the received data and information from the server and the processing resources that are configured to store, access and execute computer-readable program instructions.

The user input module is configured to control and to receive the input information from the user. Accordingly, the user input module comprises a plurality of input devices, a virtual reality input module and a virtual reality control module.

The system further comprises a user interaction module comprising a multiplayer module that is configured to facilitate a real-time interaction of the user with the system.

The user interaction module also comprises an input/output module that is configured to facilitate further interactions with the system (e.g. via a motion tracking device or a head-mounted display), and to output 2D and 3D views of the subject anatomical structures.

In particular, the input/output module comprises a rendering module, a motion tracking device, a 3D-display, a 2D-display and a head-mounted display.

The input/output module is therefore configured to display the visualizations on the 2D and 3D displays included therein, and to provide an immersive view into the anatomical structures of the patient through the head-mounted display. In particular, the immersive view is generated from the image data and the processed data, preferably using a raymarching technique.

In addition, the user interaction module is configured to create a visualization of the anatomical structures in 2D and 3D based on the image data and the processed data received from the control module. It is further configured to edit the created 2D and 3D visualizations based on the input information received from the user input module.

The user interaction module, the user input module and the input/output module are configured to communicate over a communication interface with each other and with the control module. The communication may be a wireless communication or a cabled communication.

The rendering module of the input/output module is configured to edit in real-time the 2D visualization, the 3D visualization and the immersive view based on the information received from the user interaction module, the user input devices, the virtual reality input module and the motion tracking device.

In particular, the rendering module comprises rendering algorithms, e.g. raymarching algorithms, which are configured to perform solid rendering, volume rendering and/or transparent mesh rendering of the segments of the anatomical structures. The rendering algorithms are further configured to provide stereoscopic viewings.

In particular the plurality input devices include at least one of a mouse, a joystick, a controller, a motion sensor, a keyboard and a microphone. The plurality of input devices advantageously facilitates inputting, receiving and adding various types of data and information into the system in an efficient manner.

The user input module may further include a shortcut keys (or hotkeys) module that is configured to control a list of keyboard keys which serve a general purpose, rather than a specific purpose. In particular, it is configured to provide for a layer of separation between some objects (e.g. 3D models) and the components of the user input module. The hotkeys module is further configured to provide for a location to add codes for keyboard driven events which do not belong to any specific object.

In particular, the user input module also comprises a mouse control module configured to conduct predetermined functions using the mouse and the keyboard. In this way, the input devices are functioning together to quickly conduct the predetermined functions, such as editing the opacity of an image.

The user input module may further comprise a tool manager module that is configured to manage virtual reality (VR) tools (user tools), wherein a VR tool determines a state for a VR controller. In particular, the VR tool is attached to the VR controller and is configured to perform actions based on the inputs received from the VR input module. Advantageously, the tool manager module is configured to convert actions/instructions defined by the VR controller into real actions within the VR environment.

Alternatively or additionally, the user input module further comprises a transparency plane module that is configured to create a clipping plane to determine a scene to be seen by a camera, e.g. in a viewport. This advantageously provides for a clear (and less busy) view into the subject organ.

The user input module may further include a scan slice module that is configured to control the 2D visualization of the CT scans in the VR environment.

In particular, the user interaction module comprises a segment manager module that is configured to load segments of the anatomical structures into the system, i.e. into an active workspace. In particular, the segment manager module is further configured to store a state of each segment of the anatomical structures and to create a list thereof. In this way, new segments of the anatomical structures can be loaded (added) into the system and the active workspace can be dynamically modified. The state refers to a level of transparency in which the segment is rendered, i.e. shell, solid or another percentage of transparency.

The user interaction module further comprises a path manager module that is configured to create a list of paths to be followed by the camera in the virtual reality environment and/or in the 2D environment. Advantageously, the path manager module provides for a reliable preoperative planning procedure on the basis of the predefined visualization steps defined by the paths of the camera.

In particular, the user interaction module further comprises a brush manager module that is configured to create coloured segmentations in the virtual reality environment on the basis of the input information. In this way the created segmentations in the VR environment can be easily controlled and differentiated by the user by using different colours. Preferably, the brush manager module is further configured to provide for a list of coloured (brushed) segmentations and to save said list of coloured segmentations.

The user interaction module further comprises a preset manager module including preconfigured arrays of segment settings for delineating different viewpoints for available segments (or segmentation data). Advantageously, in this way, specific viewpoints for the available segmentations can quickly and automatically be delineated for the user.

The preconfigured arrays of segment settings comprise image data, auto segmentation, broncho vascular, arteries, veins, airways, segments (solid), segments (shells), full anatomy.

Further, the user interaction module may also comprise an auto-segmentation module that is configured to create a new segment of the anatomical structure and to add the same into the active workspace. Advantageously, the auto-segmentation manager module automatically creates the new segments using software tools.

In particular, alternatively or additionally, the user interaction module comprises a workspace control module that is configured to create files and a list of paths that can be linked to a new workspace.

In particular, the workspace control module is configured to control and to modify workspaces within the system before the workspaces are loaded. In this way the files can be quickly loaded to the corresponding workspace and the therewith associated paths can reliably be linked to the workspace.

For example, a workspace may include a combination of a patient's CT-scan, segmentations, measurements that may be saved automatically. The system is configured to create new workspaces by loading a new CT-scan with, if preferred other segmentations.

In particular, the input/output module may further comprise a scan container module that is configured to control the features or characteristics of the scan objects. In particular, the scan container module is configured to control positioning, scaling and rotation of the anatomical structures that are displayed. In this way the features of the displayed anatomical structures, i.e. the scan objects, can be individually modified as required.

Additionally or alternatively, the input/output module may further comprise a screenshot module that is configured to save a screen capture of a workspace. Advantageously, the properties of the captured frame can be defined to create an output image with a high resolution. Also meta data can be added into the output image.

In particular, the data storage devices of the control module include a video memory, RAM, ROM and flash memory.

In particular, the control module may further comprise an authorization module configured to that is configured to implement an authorization functionality, such that only authorized users have access. For example, a unique code for a software installation included (saved) in the system . Based on an internal system's code, an access key is provided which is unique, authorizes the user and grants access through the control module.

In particular, the authorization module advantageously functions to prevent users to copy and paste a software built on another computer. By having the connected unique code per computer, an unauthorized duplication of the technology is prevented.

Alternatively or additionally, the control module may comprise an auto-saving module that is configured to automatically save workspaces. Advantageously, the saved workspaces can easily be controlled by the workspace control module.

In particular, the user interaction module may further comprise an event control module that is configured to convert or turn mouse clicks to button actions in an active workspace.

In addition, the user interaction module may further comprise a measurement control module that is configured to control and to create measurements and procedures to be conducted by the system (e.g. using 2D tools or 3D (VR) tools). Preferably, each measurement includes a name, value, a visualization in 2D and 3D, and lists of coordinates. This in turn results in a line between two decided coordinates with a quantitative measure in millimetres.

In particular, the measurement control module is configured to compute the value of the created measurements based on the current coordinates thereof. Alternatively or additionally, the measurement control module is configured to edit the measurements and/or to trigger saving/deleting thereof.

The measurements include 2D or 3D measurements that are configured to be created via the user input module.

In particular, the measurements may further include a sphere tool and/or a marker tool that are configured to edit the CT-scans along the coordinates provided thereon. The sphere and marker tools are configured to be applied in VR and on the 2D screen.

In particular, the measurement control module is configured to keep track of the (saved) measurements in the current workspace and of the active measurement.

In particular, the measurement control module is configured to handle saving and deleting of the measurements. Preferably, the measurement control module is configured to trigger the workspace manager module for updating the workspace file.

Further, the measurement control module may trigger a measurement panel to update the list of measurements that are displayed.

For example, the events and combination of preferred CT-scan settings, colours of segments, measurements or sphere tools are configured to be saved in the workspace so that when a user closes the program and restarts the application, the preferred settings are automatically rendered.

In addition, once a workspace is loaded, the workspace control module is configured to notify the measurement control module to load the corresponding measurements from the loaded workspace.

The above interaction and communication of the measurement control module and the workspace control module provides for a reliable transfer of the image data and information within the system as well as a uniform visualisation procedure.

In particular, the system may further comprise modules for visualizing the 3D models on a phone and website through a cloud.

In particular, the system is configured to perform a method of dynamic modeling for making 3D patient images interactive.

The method of dynamic modeling describes a software pipeline that processes 3D images into interactive software. In particular, 3D images of patients are acquired from CT scans or MRI scans as a prerequisite. The method of dynamic modeling then enables the user to perform a simulated surgical procedure of the patient-specific anatomy based on the acquired 3D image. The method is intended to be used as a tool to plan procedures pre- and intra-operatively.

In particular, the method comprises the steps of:
- (optionally) acquiring 3D images of patients from CT scans or MRI scans as a prerequisite,
- labeling anatomical structures in the 3D images for defining which voxels of the original 3D patient image belong to specific anatomical structures,
- creating 3D surfaces, preferably represented by a triangle mesh, for each of the anatomical structures based on previously created labels;
- defining a volume of each anatomical structure;
- assigning physical properties to each anatomical structure;
- simulating a transition of the patient into the operative state; and
- performing an interactive simulation of the patient.

In particular, in the step of creating 3D surfaces, the created surface mesh is used to represent the physical boundary of each anatomical structure and to visually represent them in interactive 3D graphics.

In particular, the step of defining a volume of each anatomical structure creates a volumetric representation of each of the anatomical structures. The volumetric representation consists of a mesh of tetrahedra, where a tetrahedron is a 3D primitive shape. This volumetric mesh defines the object that is being simulated.

In particular, the step of assigning is based on a set of presets properties which is stored in the software. The user then selects one of the presets that best matches the health condition of the patient in question. Thereafter, the transition of the patient into the operative state is stimulated.

In particular, the operative state involves a new pose of the patient during an operation and the effects of incisions. The effects of the initial incision of an operation are simulated using the finite element method. Accordingly, the finite element deforms the anatomical structures conformal to the predicted forces during the transition to the operative state. Advantageously, the stimulation is performed in an interactive and accurate manner.

In particular, in the step of preforming simulation, the simulation runs in an interactive 3D environment where the user can interact and the result is visually rendered in real-time. The finite element method may further be used to simulate the flexible tissue of the patient.

In particular, the method facilities the interaction of the user by performing surgical maneuvers via a computer mouse or motion-tracking controllers. The simulation loop then simulates the forces of these maneuvers onto the anatomical structures and renders the deformed state of the anatomical structure to a display in real-time.

The present invention will be better understood with the aid of the description of an embodiment given by way of example and illustrated by the figures.

It is shown in:
- **Fig. 1**: a schematic view of the system according to the present invention;
- **Fig. 2**: a schematic view of the user input module;
- **Fig. 3**: a schematic view of the user interaction module;
- **Fig. 4**: a schematic view of the user input/output module;
- **Fig. 5**: an illustrative view of the performance of a sphere tool;
- **Fig. 6**: an illustrative view of the performance of a marker tool;
- **Fig. 7**: a flow-chart of a method of dynamic modeling for making 3D patient images interactive; and
- **Fig. 8**: a flow-chart of the possible algorithm for the system.

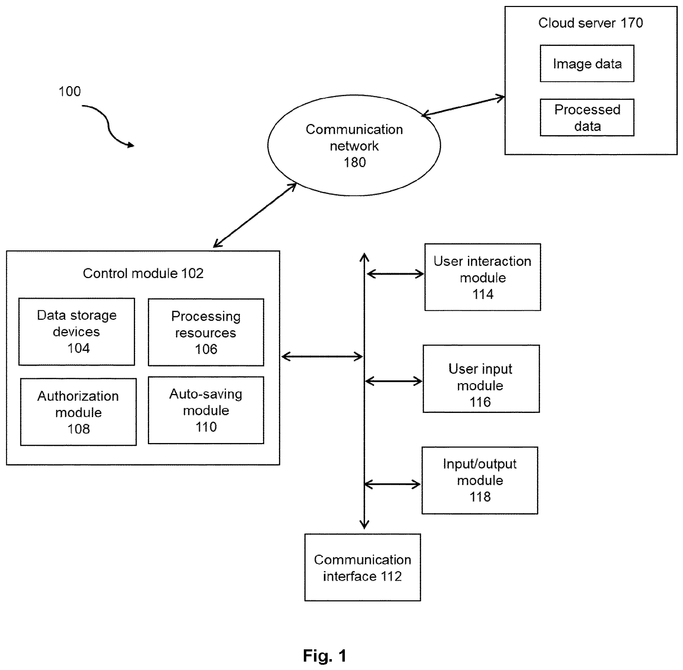

**Fig. 1** schematically illustrates a system 100 for visualising organs for planning of resections of tumors prior to surgery and for providing an immersive view into anatomical structures of a patient. The system 100 comprises a control module 102 that is communicating over a communication network 180 with a cloud server 170.

The server 170 comprises medical imagining (e.g. with DICOM, NIFTI or OBJ extensions), such as 2D CT scan images and processed images, i.e. 3D images that are e.g. generated using AI-based algorithms from the 2D CT scan images.

The system 100 further comprises a user interaction module 114, a user input module 114 and an input/output module 118 communicating with each other and with the control module 102 via a communication interface 112. The communication may include a wireless or cabled communication.

The control module 102 comprises data storage devices and processing resources 106.

The data storage devices 104, for example, include a video memory, RAM, ROM and flash memory.

The processing resources 106 include, for example, Intel core i7 (or faster processors) that are configured to perform instructions stored in the storage devices

In addition, the control module 102 may further comprise an authorization module 108 that is configured to implement an authorization check functionality for validating whether the correct authorization key is in place, such that only authorized users have access to the system.

Also it is envisaged that the control module102 to include an auto-saving module 110 that is configured to automatically save the workspaces. For example, the automatic saving of the workspace can be saved every 3, 5, 7 and/or 5 seconds. Advantageously, the saved workspaces can be controlled with a "Quick Load" option as described in the following.

The system 100 may further comprise modules (not shown) that are configured to transfer 3D renders (3D models of the anatomical structures) on e.g. a mobile phone, a tablet and/or a web browser. In this way, the system advantageously provides for mobile 2D and/or 3D visualizations.

In addition, the system 100 may comprise a remote display device, preferably a 2D-dispay and/or a 3D-display for viewing the 3D models and 3D renders during a surgical procedure.

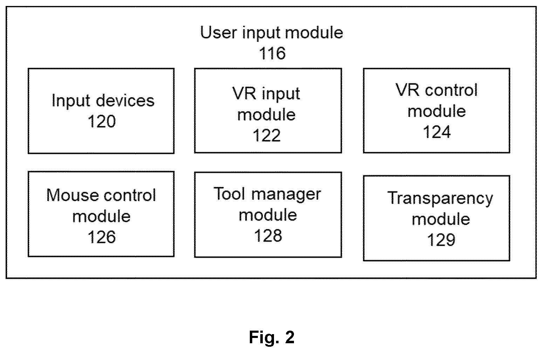

**Fig. 2** schematically illustrates the components of the user input module 116. Accordingly, the user input module 116 comprises a plurality of input devices 120, a virtual reality (VR) input module 122, a VR control module 124 and a mouse control module 126.

The user input module 116 may further comprise a tool manager module 128 that is configured to manage or control VR tools (or user tools) of the VR input module 122.

The VR tools, each are attached to the VR control module 124 (the VR controller) and are configured to perform actions based on the user inputs received from the VR input module 122. The tool manager module 128 preferably is configured to initialize a (predefined) list of user tools. In this way, actions/instructions defined by the VR controller 124 can be effectively converted into real actions within the VR environment, e.g. the MedicalVR software.

The mouse control module 126 is configured to control special behaviour for the mouse cursor in the software application, when it is not behaving like a cursor. For example, the user can hold a keyboard key and move the mouse to directly control the opacity of the image.

The user input module 116 may further include a hotkeys module (not shown). This module is configured to provide a layer of separation between some objects and the user input module 116, as well as to provide a location to add code for keyboard driven events which do not belong to any specific object.

For example, the short keys may be predefined to trigger the following actions:
- Select scan preset
- Reset position and all options
- Reset position
- Reset segments transparency and reset colours
- Toggle instant render update
- Change stereoscopic mode
- Toggle segment colouring
- Rotate the 3D-model
- Zoom in or out

In addition, the user input module 116 may further comprise a transparency plane module 129 which, for example, includes a slicing tool to create a clipping plane. The transparency plane module 129 is configured to determine how much of a scene is seen by a camera in a viewport. Segments of the anatomical structures and scan data that are occluded by the transparency plane module 129 will not be rendered, which allows the user to easily see into the subject organ.

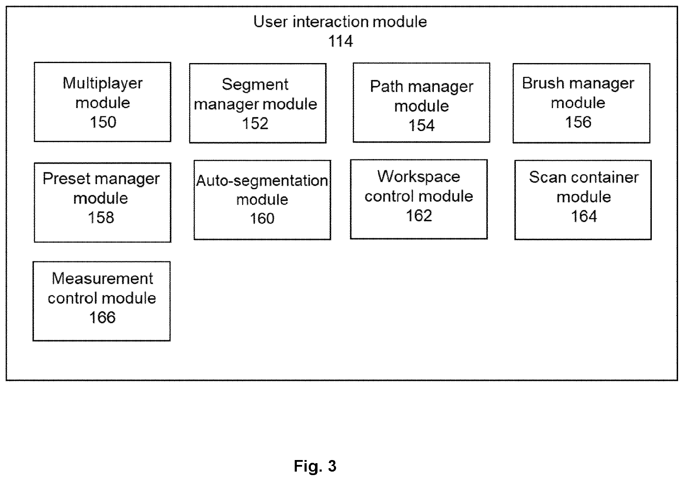

A scan slice module (not shown) may also be included in the user input module 116 that is configured to control the 2D visualization of the CT scans in the VR environment, e.g. the MedicalVR software. **Fig. 3** schematically illustrates the components of the user interaction module 114. The user interaction module 114 comprises a multiplayer module 150, a segment manager module 152, a path manager module 154 and a brush manager module 156.

The multiplayer module 150 is configured to allow real-time interactions with (and/or modifications of) the scan images and the viewings. It is further configured to facilitate the manipulation and variation of scan views and drawings in real time while, for example, enabling voice chat capabilities to simulate a live session together with other remote users.

The multiplayer module 150 may therefore be configured to perform the following functions in real time between users by, e.g. transmitting:
- Users' head and hand controller positional data
- Live voice audio (not recorded, only streamed)
- Scan pan/rotation/scale
- Scan preset state
- Scan visual settings
- Pen and brush drawings
- Any other basic workspace data or settings

Identifiers of users and host/guest permissions in controlling the sessionThe segment manager module 152 is configured to manage and control the segments of the anatomical structures which can be loaded into a workspace. This module is further configured to store the state of each segment and to create a list of the (loaded) segments. The segment manager module 152 advantageously facilitates to add new segments or remove segments from the current (active) workspace.

Further, the path manager module 154 of the user interaction module 114 is configured to create a list of paths that is to be followed by a camera in the virtual reality environment and/or in the 2D environment. Accordingly, the path of the camera can be predetermined and/or adjusted by the user.

In particular, the brush manager module 156 is configured to create coloured segmentations in the virtual reality environment on the basis of the user input information. The so-called brush is actually a created colouring of a segment in the virtual environment by the user. In this way, the user can create a brush segmentation by adding colours in the CT scans.

The brush manager module 156 may further be configured to provide for a list of brush segmentations and to save said list.

A preset manager module 158 is also included in the user interaction module 114 for controlling the presets in the workspaces. These presets are preconfigured arrays of segment settings to delineate different viewpoints for available scan segmentations in a quick and easy manner for the user.

The presets, for example, include the original CT scans, auto-segmentations, broncho-vascular, arteries, veins, airways, segments (solid), segments (shells) and full anatomy. Solid segments are not see through, shell segments are see through and have about 20-30% of the transparency of a solid segment.

Additionally or alternatively, the user interaction module 114 may comprise an auto-segmentation module 160 that is configured to (automatically) create new segments of the anatomical structures using software tools. The new segments are configured to be loaded into the active workspace.

In particular, the auto-segmentation module 160 is further configured to store the state of each new segment and to create a list of the new segments.

The user interaction module 114 may further comprise a workspace control module 162 that is configured to control workspaces before they are loaded. Preferably, the workspace control module 162 is configured to create files and a list of paths that can be linked to a new workspace. For example, the workspace control module 162 is configured to control the loading of the files and to create a list of recent files which can be used for the so-called "Quick Load" option in the MedicalVR software.

The user interaction module 114 may further comprise a measurement control module 166 which is configured to control and create measurements and procedures to be conducted using the system 100. Each measurement includes a name, value, a visualization in 2D and 3D, and lists of coordinates. Every type of measurement (e.g. Distance, Sphere tool or Marker tool) is a subclass of measurements. The measurements, spheres or markers are saved in the workspace with accompanied coordinates. When a measurement is created or edited, the measurement control module 166 is configured to compute its value based on its current coordinates, and to create (or to update) the corresponding visualizations accordingly. The measurement control module 166 is further configured to edit the measurements and/or to trigger saving/deleting thereof.

The measurements include 2D or 3D measurements that are configured to be created via the user input module 116. For example, the 2D measurements are created based on the mouse input of the user.

The measurement control module 166 is further configured to keep track of the (saved) measurements in the current workspace and of the one that is currently loaded by the user (i.e. the active measurement).

In particular, the measurement control module 166 is configured to handle saving and deleting of the measurements. For example, when a measurement is saved or deleted, the measurement control module 166 is configured to trigger the workspace manager module 162 to update the workspace file and a measurement panel to update its displayed list of measurements.

In particular, when the user loads a workspace, the workspace control module 162 is configured to notify the measurement control module 166 to load in the corresponding measurements from the loaded workspace.

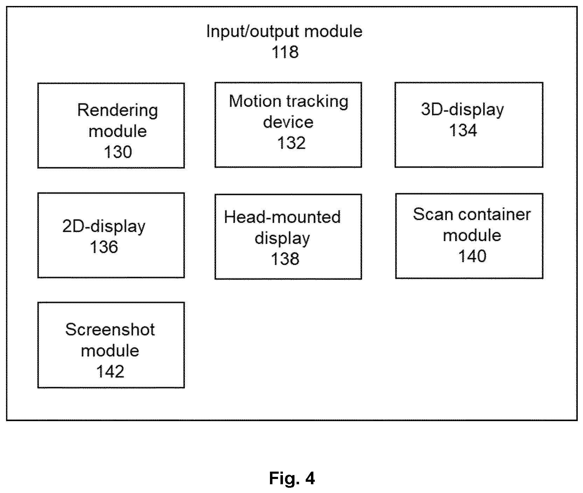

**Fig. 4** schematically illustrates the components of the input/output module 118. The input/output module comprises a rendering module 130, a motion tracking device 132, and 2D and 3D displays 136, 138.

The rendering module 130 comprises rendering algorithms, e.g. raymarching algorithms, which are is configured to perform solid rendering, volume rendering and/or transparent mesh rendering of the segments. It is further configured to provide a stereoscopic display.

The motion tracking device 132 comprises sensors, e.g. for capturing data on the position, orientation and velocity of the hands or body.

The 2D and 3D displays 136, 138, for example, include standard computer displays and stereoscopic 3D HDMI displays, respectively.

The input/output module 118 may alternatively or additionally comprise a scan container module 140. In particular, the scan container module 140 is configured to control the positioning, scaling and rotation of the scan objects. The scan container module 140 is further configured to automatically convert different units such as voxels, scene units and millimetres.

In particular, the input/output module 118 may also comprise a screenshot module 142 that is configured to save a screen capture of the workspace. Preferably, before making a given screenshot, the settings are set to a high level for one frame to create an image with high resolution. Meta data can also optionally be inserted into the output image.

It is further envisaged that the system comprises modules enabling a surgeon(s) to view renders, 3D models of the anatomical structures during a given surgical procedure.

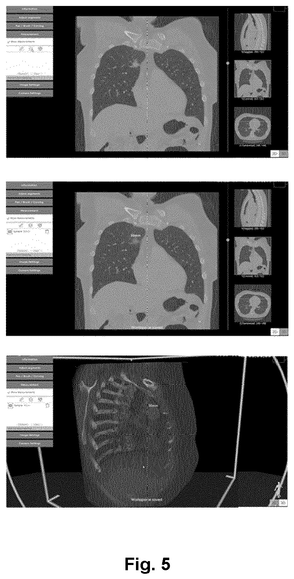

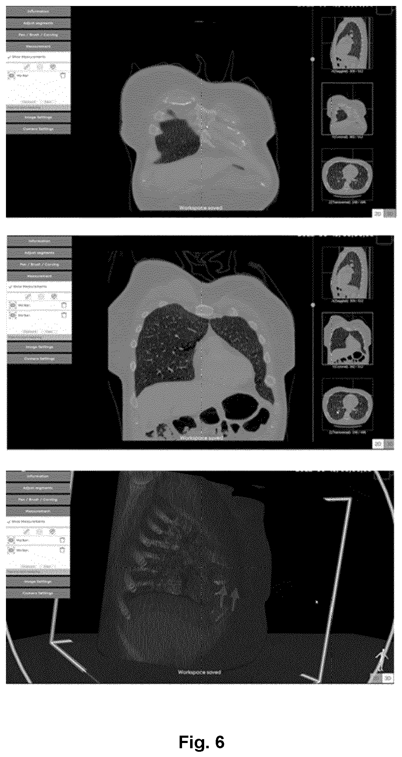

**Figs. 5** and **6** illustratively show the performance of the sphere tool and the marker tool of the system 100, respectively. The sphere tool is configured such that a user can create a 3D sphere around a specific anatomical area of interest. Once the sphere tool is triggered in 2D or 3D views of the anatomical structures (the top panel in **Fig. 5**), the user is able to point and click on the area of interest. Thereafter, the user can click and drag to a preferred size to define a sphere (the middle panel in **Fig. 5**) and view the area in the 3D-view. As the center of the sphere is calculated as starting point, the size is based on the radius. In 2D-view the circle is drawn, while in 3D-view a 3D sphere is visualized. Similarly, the marker tool is configured such that the user can mark an area of interest in e.g. a 2D-view and then view that marked area in the 3D-view as indicated in **Fig. 6** from the top to bottom panels, respectively.

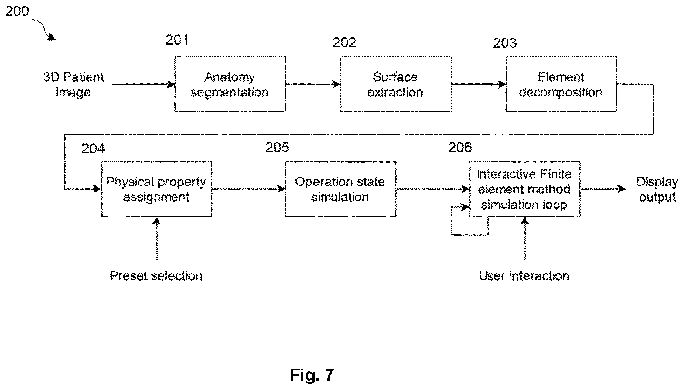

**Fig. 7** illustrates a method 200 of dynamic modeling for making 3D patient images interactive. The method is advantageously performed by the system 100.

The 3D patient image may optionally be fed through the pipeline illustrated in **Fig. 7**.

At step 201 the anatomical structures in the 3D image are labeled. This step defines which voxels of the original 3D patient image belong to specific anatomical structures. At step 202 3D surfaces are created for each anatomical structure based on the previously created labels. The 3D surfaces are represented by a triangle mesh. This surface mesh is used to represent the physical boundary of each anatomical structure and to visually represent them in interactive 3D graphics.

Then at step 203 a volume of each anatomical structure is defined. The volumetric representation consists of a mesh of tetrahedra, where a tetrahedron is a 3D primitive shape. The volumetric mesh defines the object that is being simulated.

At step 204 the physical properties are assigned to each anatomical structure. This is based on a set of presets of properties which is stored in the software. The user then selects one of the presets that best matches the health condition of the patient in question.

At step 205 the transition of the patient into the operative state is stimulated. The operative state involves a new pose of the patient during an operation and the effects of incisions. The effects of the initial incision of an operation are simulated using the finite element method. The finite element method in this software deforms the anatomical structures conformal to the predicted forces during the transition to the operative state.

Finally, at step 206 an accurate and interactive simulation of the patient is performed. This simulation runs in an interactive 3D environment where the user can interact and the result is visually rendered in real-time. The finite element method can be used again to simulate the flexible tissue of the patient.

Advantageously, the step 206 may be run on a local computer with high-end graphics capabilities. The user interacts by performing surgical maneuvers via a computer mouse or motion-tracking controllers. The simulation loop then simulates the forces of these maneuvers onto the anatomical structures and then renders the deformed anatomical state to a display in real-time.

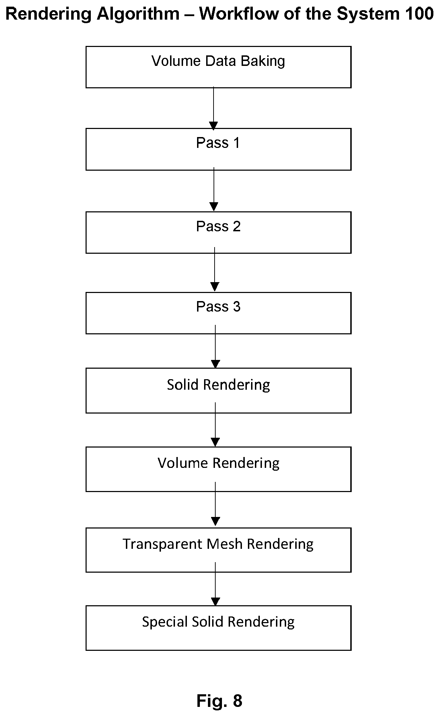

The rendering algorithm could be as follows (cf. **Fig. 8**):

### Rendering algorithm for the main image:

Volume data baking
If settings or data have changed, refresh the cached voxel bitmaps (Compute shader)
(This is done as infrequently as possible, because it's expensive.)
Clear the voxel and segment data texture buffers.

### Pass 1

Rebuild the data texture by parsing segments in order from least to most important.

Segments can overlap, but the highest one overwrites voxels from lower priority segments.

User made brushes are treated like segments, but they are stored differently for efficiency.

Mesh based segments cannot be handled here, they are drawn as unrelated 3D objects.

This step has created a 3D bitmap containing indexes instead of colors, referencing a different array which contains color/opacity/fill/etc.

### Pass 2

Combines the processed segment data with the scan data.

This parses every raw voxel and outputs it to another voxel buffer.

The algorithm for this is pretty detailed, but also still changes frequently.

It decides which voxels should be completely filtered out, and the transparency of those which remain.

Output voxels get their color from the segment, or from the shading texture (if provided).

If the segment has "fill" on, the above transparency choices are overriden here.

### Pass 3

Applies an edge detecting filter for the entire volume, based on per-segment settings.

For applicable segments, the transparency of edge pixels is increased, and decreased voxels is decreased.

Note: This step will probably be moved to pass 1, depending on what behavior is prefered.

### Solid rendering

Draw every solid object which should be affected by the scan image.

Note: The depth buffer from this stage needs to be accessible for the volume rendering stage.

### Volume rendering

For each visible screen pixel, process a ray through the voxel bitmap. (Frag shader)
Account for the Transparency Plane when calculating the start/end points of the ray.

Calculate the number of ray samples to be taken based on settings (typically 500-1000).

Combine each sampled voxel using front to back ordering.

Stop processing the ray when it collides with a solid object other than the scan content.

Currently done by using the depth buffer, and assuming all other objects are opaque.

Adjust the pixel's saturation, contrast, and brightness based on settings.

### Transparent mesh rendering (segments)

Draw all meshes, storing the frontmost alpha and depth position
Draw each fragment, using the frontmost mesh at the correct alpha and blending one/one
Add to each fragment, using every NON frontmost mesh, approximating the alpha, and blending OneMinusDstAlpha/One
Note: Accounting for the slicing tool at this stage is difficult, and will require a rewrite.

### Special solid rendering

Draw any objects which must always be visible, such as controllers
The system now renders the images as normal (VR or 2D). Stereoscopic options are also available for 2D, but do not affect the above rendering.

### Reference numerals

- 100: System
- 102: Control module
- 104: Data storage devices
- 106: Processing resources
- 108: Authorization module
- 110: Auto-saving module
- 112: Communication interface
- 114: User interaction module
- 116: User input module
- 118: Input/output module
- 120: Input devices
- 122: VR input module
- 124: VR control module
- 126: Mouse control module
- 128: Tool manager module
- 129: Transparency module
- 130: Rendering module
- 132: Motion tracking module
- 134: 3D-display
- 136: 2D-display
- 138: Head-mounted display
- 140: Scam container module
- 142: Screenshot module
- 150: Multiplayer module
- 152: Segment manger module
- 154: Path manager module
- 156: Brush manager module
- 158: Preset manager module
- 160: Auto-segmentation module
- 162: Workspace control module
- 164: Scan container module
- 166: Measurement control module
- 170: (Cloud) server
- 180: Communication network
- 200: Method
- 201: Method step
- 202: Method step
- 203: Method step
- 204: Method step
- 205: Method step
- 206: Method step

## Claims

1. A system (100) for visualising organs for planning of segmentectomy and/or lobectomy to resect tumors prior to surgery, and for providing an immersive view into anatomical structures of a patient, the system (100) comprising:
a control module (102) comprising data storage devices (104) and processing resources (106) configured to store, access and execute computer-readable program instructions;
a server (170) communicating over a communication network (180) with the control module (102);
a user interaction module (114) comprising a multiplayer module (150) configured to facilitate a real-time interaction of a user with the system (100);
a user input module (116) configured to control and to receive input information from the user, wherein the user input module (116) comprises a plurality of input devices (120), a virtual reality input module (122) for receiving input in a virtual reality environment, and a virtual reality control module (124) for conducting operations based on the information received from the virtual reality input module (122); and
an input/output module (118) comprising a rendering module (130), a motion tracking device (132), a 3D-display (134), a 2D-display (136), and a head-mounted display (138) for an immersive viewing;
the user interaction module (114), the user input module (116) and the input/output module (118) communicating over a communication interface (112) with each other and with the control module (102);
the control module (102) configured to receive image data and processed data from the server (170), and to store the same in the data storage devices (104), wherein the image data comprise 2D computed tomography images of the anatomical structures, and wherein the processed data comprise segmentation data representative of segments of the anatomical structures;
the user interaction module (114) configured to create visualizations of the anatomical structures in 2D and 3D based on the image data and the processed data received from the control module (102), and to edit the created 2D and 3D visualizations based on the input information received from the user input module (116);
wherein the user interaction module (114) further comprises a preset manager module (158) including preconfigured arrays of segment settings for delineating viewpoints for the available segmentation data, and wherein the preconfigured arrays of segment settings comprise the image data, auto segmentation, broncho vascular, arteries, veins, airways, solid segments, shell segments and full anatomy,
the input/output module (118) further configured to display the 2D visualization on the 2D-display (136) and the 3D visualization on the 3D-display (134), and to provide an immersive view into the anatomical structures of the patient through the head-mounted display (138), wherein the immersive view is generated from the image data and the processed data, preferably using a raymarching technique;
the input/output module (118) further configured to edit, using the rendering module (130), the 2D visualization, the 3D visualization and the immersive view based on the information received from the user interaction module (114), the user input devices (120), the virtual reality input module (122) and the motion tracking device (132).

2. The system (100) of claim 1,
wherein the system (100) further comprises:
another display which is remote to the system (100), wherein this display comprises 2D-dispaly and a 3D-display that are configured to display the 2D and 3D visualisations of the anatomical structures, and/or
modules configured to transfer the 2D and 3D visualisations on a mobile device.

3. The system (100) of claim 1 or 2,
wherein the plurality input devices (120) includes at least one of a mouse, a joystick, a controller, a motion sensor, a keyboard and a microphone, preferably the plurality input devices (120) includes a mouse and the user input module (116) further comprises a mouse control module (126) configured to conduct predetermined functions using the mouse and the keyboard.

4. The system (100) of any of claims 1 to 3,
wherein the user input module (116) further comprises a tool manager module (128) configured to manage virtual reality tools and/or a transparency plane module (129) configured to create a clipping plane to determine a scene to be seen by a camera.

5. The system (100) of any of the preceding claims,
wherein the user interaction module (114) further comprises a segment manager module (152) configured to load a segment of the anatomical structures into the system, preferably the segment manager module (152) is further configured to store a state of each segment of the anatomical structures and to create a list thereof.

6. The system (100) of any of the preceding claims,
wherein the user interaction module (114) further comprises a path manager module (154) configured to create a list of paths to be followed by a camera in the virtual reality environment and/or in the 2D environment.

7. The system (100) of any of the preceding claims,
wherein the user interaction module (114) further comprises a brush manager module (156) configured to create coloured segmentations in the virtual reality environment based on the input information.

8. The system (100) of any of the preceding claims,
wherein the user interaction module (114) further comprises an auto-segmentation module (160) configured to create new segments of the anatomical structures using software tools.

9. The system (100) of any of the preceding claims,
wherein the user interaction module (114) further comprises a workspace manager module (162) configured to create files and a list of paths that can be linked to a new workspace.

10. The system (100) of any of the preceding claims,
wherein the input/output module (118) further comprises a scan container module (140) configured to control positioning, scaling and rotation of the anatomical structures that are displayed.

11. The system (100) of any of the preceding claims,
wherein the input/output module (118) further comprises a screenshot module (142) configured to save a screen capture of a workspace.

12. The system (100) of any of the preceding claims,
wherein the data storage devices (104) include a video memory, RAM, ROM and flash memory.

13. The system (100) of any of the preceding claims,
wherein the control module (102) further comprises an authorization module (108) configured to authorize users to gain access to the software installation and/or an auto-saving module (110) configured to automatically save workspaces.

14. The system (100) of any of the preceding claims,
wherein the user interaction module (114) further comprises a measurement control module (166) configured to create measurements to be conducted by the system (100).

## Patentansprüche

1. Ein System (100) zur Visualisierung von Organen zur Planung einer Segmentektomie und/oder Lobektomie zur Resektion von Tumoren vor einer Operation sowie zur Bereitstellung einer immersiven Ansicht der anatomischen Strukturen eines Patienten, wobei das System (100) aufweist:
ein Steuermodul (102) mit Datenspeichervorrichtungen (104) und Verarbeitungsressourcen (106), die zum Speichern, Abrufen und Ausführen computerlesbarer Programmanweisungen ausgelegt sind;
einen Server (170), der über ein Kommunikationsnetzwerk (180) mit dem Steuermodul (102) kommuniziert;
ein Benutzerinteraktionsmodul (114), das ein Multiplayer-Modul (150) umfasst, das so konfiguriert ist, dass es eine Echtzeit-Interaktion eines Benutzers mit dem System (100) ermöglicht;
ein Benutzereingabemodul (116), das so konfiguriert ist, dass es Eingaben des Benutzers steuert und empfängt, wobei das Benutzereingabemodul (116) eine Vielzahl von Eingabegeräten (120), ein Virtual-Reality-Eingabemodul (122) zum Empfangen von Eingaben in einer Virtual-Reality-Umgebung und ein Virtual-Reality-Steuermodul (124) zum Ausführen von Operationen auf der Grundlage der vom Virtual-Reality-Eingabemodul (122) empfangenen Informationen umfasst; und
ein Ein-/Ausgabemodul (118), das ein Rendering-Modul (130), eine Bewegungserfassungsvorrichtung (132), ein 3D-Display (134), ein 2D-Display (136) und ein Head-Mounted-Display (138) für eine immersive Betrachtung umfasst;
wobei das Benutzerinteraktionsmodul (114), das Benutzereingabemodul (116) und das Ein-/Ausgabemodul (118) über eine Kommunikationsschnittstelle (112) miteinander und mit dem Steuermodul (102) kommunizieren;
das Steuermodul (102) ist so konfiguriert, dass es Bilddaten und verarbeitete Daten vom Server (170) empfängt und diese in den Datenspeichervorrichtungen (104) speichert, wobei die Bilddaten 2D-Computertomographiebilder der anatomischen Strukturen umfassen und wobei die verarbeiteten Daten Segmentierungsdaten umfassen, die Segmente der anatomischen Strukturen darstellen;
das Benutzerinteraktionsmodul (114) ist so konfiguriert, dass es auf der Grundlage der vom Steuermodul (102) empfangenen Bilddaten und verarbeiteten Daten Visualisierungen der anatomischen Strukturen in 2D und 3D erstellt und die erstellten 2D- und 3D-Visualisierungen auf der Grundlage der vom Benutzereingabemodul (116) empfangenen Eingabeinformationen bearbeitet;
wobei das Benutzerinteraktionsmodul (114) ferner ein Voreinstellungs-Manager-Modul (158) umfasst, das vorkonfigurierte Arrays von Segment-Einstellungen zur Abgrenzung von Blickwinkeln für die verfügbaren Segmentierungsdaten enthält, und wobei die vorkonfigurierten Arrays von Segment-Einstellungen die Bilddaten, die automatische Segmentierung, die broncho-vaskulären Strukturen, Arterien, Venen, Atemwege, solide Segmente, Schalen-Segmente und die vollständige Anatomie umfassen,
wobei das Eingabe-/Ausgabemodul (118) ferner so konfiguriert ist, dass es die 2D-Visualisierung auf dem 2D-Display (136) und die 3D-Visualisierung auf dem 3D-Display (134) anzeigt, sowie dazu, über das Head-Mounted-Display (138) eine immersive Ansicht der anatomischen Strukturen des Patienten bereitzustellen, wobei die immersive Ansicht aus den Bilddaten und den verarbeiteten Daten, vorzugsweise unter Verwendung einer Raymarching-Technik, erzeugt wird;
wobei das Eingabe-/Ausgabemodul (118) ferner so konfiguriert ist, dass es unter Verwendung des Rendering-Moduls (130) die 2D-Visualisierung, die 3D-Visualisierung und die immersive Ansicht auf der Grundlage der Informationen bearbeitet, die vom Benutzerinteraktionsmodul (114), den Benutzereingabegeräten (120), dem Virtual-Reality-Eingabemodul (122) und dem Bewegungserfassungsvorrichtung (132) empfangen werden.

2. Das System (100) nach Anspruch 1,
wobei das System (100) ferner umfasst:
ein weiteres, vom System (100) entferntes Display, wobei dieses Display ein 2D-Display und ein 3D-Display umfasst, die dazu ausgelegt sind, die 2D- und 3D-Visualisierungen der anatomischen Strukturen anzuzeigen, und/oder
Module, die so konfiguriert sind, dass sie die 2D- und 3D-Visualisierungen auf ein mobiles Gerät übertragen.

3. Das System (100) nach Anspruch 1 oder 2,
wobei die mehreren Eingabegeräte (120) mindestens eines der folgenden Elemente umfassen: eine Maus, einen Joystick, einen Controller, einen Bewegungssensor, eine Tastatur und ein Mikrofon; vorzugsweise umfassen die mehreren Eingabegeräte (120) eine Maus, und das Benutzereingabemodul (116) umfasst ferner ein Maussteuerungsmodul (126), das so konfiguriert ist, dass es unter Verwendung der Maus und der Tastatur vorbestimmte Funktionen ausführt.

4. Das System (100) nach einem der Ansprüche 1 bis 3,
wobei das Benutzereingabemodul (116) ferner ein Werkzeugverwaltungsmodul (128), das zur Verwaltung von Virtual-Reality-Werkzeugen ausgelegt ist, und/oder ein Transparenzebenenmodul (129) umfasst, das zur Erzeugung einer Ausschnittsebene ausgelegt ist, um eine von einer Kamera zu erfassende Szene zu bestimmen.

5. Das System (100) nach einem der vorstehenden Ansprüche,
wobei das Benutzerinteraktionsmodul (114) ferner ein Segmentverwaltungsmodul (152) umfasst, das zum Laden eines Segments der anatomischen Strukturen in das System ausgelegt ist, wobei das Segmentverwaltungsmodul (152) vorzugsweise ferner dazu ausgelegt ist, einen Zustand jedes Segments der anatomischen Strukturen zu speichern und eine Liste davon zu erstellen.

6. Das System (100) nach einem der vorstehenden Ansprüche,
wobei das Benutzerinteraktionsmodul (114) ferner ein Pfadverwaltungsmodul (154) umfasst, das so konfiguriert ist, dass es eine Liste von Pfaden erstellt, denen eine Kamera in der Virtual-Reality-Umgebung und/oder in der 2D-Umgebung folgen soll.

7. Das System (100) nach einem der vorstehenden Ansprüche,
wobei das Benutzerinteraktionsmodul (114) ferner ein Pinselverwaltungsmodul (156) umfasst, das so konfiguriert ist, dass es auf der Grundlage der Eingabeinformationen farbige Segmentierungen in der Virtual-Reality-Umgebung erstellt.

8. Das System (100) nach einem der vorstehenden Ansprüche,
wobei das Benutzerinteraktionsmodul (114) ferner ein Auto-Segmentierungsmodul (160) umfasst, das so konfiguriert ist, dass es mithilfe von Software-Tools neue Segmente der anatomischen Strukturen erstellt.

9. Das System (100) nach einem der vorstehenden Ansprüche,
wobei das Benutzerinteraktionsmodul (114) ferner ein Arbeitsbereichsverwaltungsmodul (162) umfasst, das so konfiguriert ist, dass es Dateien und eine Liste von Pfaden erstellt, die mit einem neuen Arbeitsbereich verknüpft werden können.

10. Das System (100) nach einem der vorstehenden Ansprüche,
wobei das Eingabe-/Ausgabemodul (118) ferner ein Scan-Container-Modul (140) umfasst, das so konfiguriert ist, dass es die Positionierung, Skalierung und Drehung der angezeigten anatomischen Strukturen steuert.

11. Das System (100) nach einem der vorstehenden Ansprüche,
wobei das Eingabe-/Ausgabemodul (118) ferner ein Screenshot-Modul (142) umfasst, das so konfiguriert ist, dass es eine Bildschirmaufnahme eines Arbeitsbereichs speichert.

12. Das System (100) nach einem der vorstehenden Ansprüche,
wobei die Datenspeichevorrichtung (104) einen Videospeicher, einen RAM, einen ROM und einen Flash-Speicher umfassen.

13. Das System (100) nach einem der vorstehenden Ansprüche,
wobei das Steuermodul (102) ferner ein Autorisierungsmodul (108), das so konfiguriert ist, dass es Benutzern den Zugriff auf die Softwareinstallation gewährt, und/oder ein Modul zum automatischen Speichern (110), das so konfiguriert ist, dass es Arbeitsbereiche automatisch speichert, umfasst.

14. Das System (100) nach einem der vorstehenden Ansprüche,
wobei das Benutzerinteraktionsmodul (114) ferner ein Messsteuerungsmodul (166) umfasst, das so konfiguriert ist, dass es Messungen erstellt, die vom System (100) durchgeführt werden sollen.

## Revendications

1. Système (100) destiné à visualiser des organes en vue de la planification d'une segmentectomie et/ou d'une lobectomie visant à réséquer des tumeurs avant une intervention chirurgicale, et à fournir une vue immersive des structures anatomiques d'un patient, le système (100) comprenant:
un module de contrôle (102) comprenant des dispositifs de stockage de données (104) et des ressources de traitement (106) configurés pour stocker, accéder à et exécuter des instructions de programme lisibles par ordinateur;
un serveur (170) communiquant avec le module de contrôle (102) via un réseau de communication (180);
un module d'interaction utilisateur (114) comprenant un module multijoueur (150) configuré pour faciliter une interaction en temps réel d'un utilisateur avec le système (100);
un module d'entrée utilisateur (116) configuré pour contrôler et recevoir des informations d'entrée provenant de l'utilisateur, dans lequel le module d'entrée utilisateur (116) comprend une pluralité de dispositifs d'entrée (120), un module d'entrée de réalité virtuelle (122) destiné à recevoir des entrées dans un environnement de réalité virtuelle, et un module de contrôle de réalité virtuelle (124) destiné à effectuer des opérations sur la base des informations reçues du module d'entrée de réalité virtuelle (122); et
un module d'entrée/sortie (118) comprenant un module de rendu (130), un dispositif de suivi de mouvement (132), un écran 3D (134), un écran 2D (136) et un casque de réalité virtuelle (138) permettant une visualisation immersive;
le module d'interaction utilisateur (114), le module d'entrée utilisateur (116) et le module d'entrée/sortie (118) communiquant entre eux et avec le module de contrôle (102) via une interface de communication (112);
le module de contrôle (102) est configuré pour recevoir des données d'image et des données traitées provenant du serveur (170), et pour les stocker dans les dispositifs de stockage de données (104), les données d'image comprenant des images de tomodensitométrie en 2D des structures anatomiques, et les données traitées comprenant des données de segmentation représentatives de segments des structures anatomiques;
le module d'interaction utilisateur (114) est configuré pour créer des visualisations des structures anatomiques en 2D et en 3D sur la base des données d'image et des données traitées reçues du module de contrôle (102), et pour modifier les visualisations 2D et 3D créées en fonction des informations d'entrée reçues du module d'entrée utilisateur (116);
le module d'interaction utilisateur (114) comprenant en outre un module de gestion des préréglages (158) incluant des ensembles préconfigurés de paramètres de segmentation pour définir des points de vue pour les données de segmentation disponibles, et les ensembles préconfigurés de paramètres de segmentation comprenant les données d'image, la segmentation automatique, les structures broncho-vasculaires, les artères, les veines, les voies respiratoires, les segments solides, les segments de coque et l'anatomie complète,
le module d'entrée/sortie (118) étant en outre configuré pour afficher la visualisation en 2D sur l'écran 2D (136) et la visualisation en 3D sur l'écran 3D (134), et à fournir une vue immersive des structures anatomiques du patient via le casque de réalité virtuelle (138), la vue immersive étant générée à partir des données d'image et des données traitées, de préférence à l'aide d'une technique de raymarching;
le module d'entrée/sortie (118) étant en outre configuré pour modifier, à l'aide du module de rendu (130), la visualisation en 2D, la visualisation en 3D et la vue immersive sur la base des informations reçues du module d'interaction avec l'utilisateur (114), des dispositifs de d'entrée de utilisateur (120), du module de saisie de réalité virtuelle (122) et du dispositif de suivi de mouvement (132).

2. Le système (100) selon la revendication 1,
dans lequel le système (100) comprend en outre:
un autre écran distant du système (100), cet écran comprenant un affichage 2D et un affichage 3D configurés pour afficher les visualisations 2D et 3D des structures anatomiques, et/ou
des modules configurés pour transférer les visualisations en 2D et en 3D sur un appareil mobile.

3. Le système (100) selon la revendication 1 ou 2,
dans lequel la pluralité de dispositifs d'entrée (120) comprend au moins l'un parmi une souris, un joystick, une manette, un capteur de mouvement, un clavier et un microphone; de préférence, la pluralité de dispositifs d'entrée (120) comprend une souris et le module d'entrée utilisateur (116) comprend en outre un module de commande de souris (126) configuré pour exécuter des fonctions prédéterminées à l'aide de la souris et du clavier.

4. Le système (100) selon l'une quelconque des revendications 1 à 3,
dans lequel le module d'entrée utilisateur (116) comprend en outre un module de gestion des outils (128) configuré pour gérer des outils de réalité virtuelle et/ou un module de plan de transparence (129) configuré pour créer un plan de découpe afin de déterminer une scène devant être vue par une caméra.

5. Le système (100) selon l'une quelconque des revendications précédentes,
dans lequel le module d'interaction utilisateur (114) comprend en outre un module de gestion des segments (152) configuré pour charger un segment des structures anatomiques dans le système; de préférence, le module de gestion des segments (152) est en outre configuré pour stocker un état de chaque segment des structures anatomiques et pour créer une liste de ceux-ci.

6. Le système (100) selon l'une quelconque des revendications précédentes,
dans lequel le module d'interaction avec l'utilisateur (114) comprend en outre un module de gestion des trajectoires (154) configuré pour créer une liste de trajectoires à suivre par une caméra dans l'environnement de réalité virtuelle et/ou dans l'environnement 2D.

7. Le système (100) selon l'une quelconque des revendications précédentes,
dans lequel le module d'interaction avec l'utilisateur (114) comprend en outre un module de gestion des pinceaux (156) configuré pour créer des segmentations colorées dans l'environnement de réalité virtuelle sur la base des informations d'entrée.

8. Le système (100) selon l'une quelconque des revendications précédentes
dans lequel le module d'interaction avec l'utilisateur (114) comprend en outre un module de segmentation automatique (160) configuré pour créer de nouveaux segments des structures anatomiques à l'aide d'outils logiciels.

9. Le système (100) selon l'une quelconque des revendications précédentes,
dans lequel le module d'interaction avec l'utilisateur (114) comprend en outre un module de gestion d'espace de travail (162) configuré pour créer des fichiers et une liste de chemins d'accès pouvant être associés à un nouvel espace de travail.

10. Le système (100) selon l'une quelconque des revendications précédentes,
dans lequel le module d'entrée/sortie (118) comprend en outre un module de conteneur de numérisation (140) configuré pour contrôler le positionnement, la mise à l'échelle et la rotation des structures anatomiques affichées.

11. Le système (100) selon l'une quelconque des revendications précédentes,
dans lequel le module d'entrée/sortie (118) comprend en outre un module de capture d'écran (142) configuré pour enregistrer une capture d'écran d'un espace de travail.

12. Le système (100) selon l'une quelconque des revendications précédentes,
dans lequel les dispositifs de stockage de données (104) comprennent une mémoire vidéo, une mémoire RAM, une mémoire ROM et une mémoire flash.

13. Le système (100) selon l'une quelconque des revendications précédentes,
dans lequel le module de contrôle (102) comprend en outre un module d'autorisation (108) configuré pour autoriser les utilisateurs à accéder à l'installation du logiciel et/ou un module de sauvegarde automatique (110) configuré pour sauvegarder automatiquement les espaces de travail.

14. Le système (100) selon l'une quelconque des revendications précédentes,
dans lequel le module d'interaction avec l'utilisateur (114) comprend en outre un module de contrôle des mesures (166) configuré pour créer des mesures devant être effectuées par le système (100).
